# EUROPEAN PATENT SPECIFICATION

(11) **EP 2 332 613 B1**
(45) Date of publication and mention of the grant of the patent: **24.04.2013**
(21) Application number: 09834885.7
(22) Date of filing: 22.12.2009
(51) Int. Cl.: A61N 5/06, A45D 26/00

(54) **LIGHT IRRADIATION BEAUTY DEVICE**
LICHTSTRAHLUNGS-KOSMETIKVORRICHTUNG
DISPOSITIF DE BEAUTÉ PAR IRRADIATION LUMINEUSE

(30) Priority: 25.12.2008 JP 2008331357; 25.12.2008 JP 2008331358; 25.12.2008 JP 2008331359
(43) Date of publication of application: 15.06.2011
(73) Proprietor: Panasonic Corporation, Osaka 571-8501 (JP)
(72) Inventor: TAKADA, Kosaku, Osaka 540-6207 (JP); SUEYOSHI, Hidekazu, Osaka 540-6207 (JP); IBUKI, Yasuo, Osaka 540-6207 (JP); ASAKAWA, Koji, Osaka 540-6207 (JP); YAMASHITA, Mikihiro, Osaka 540-6207 (JP)
(74) Representative: Appelt, Christian W.
(86) International application number: PCT/JP2009/071333
(87) International publication number: WO 2010/074084

(56) References cited:
- JP-A- 2004 321 401
- JP-A- 2005 278 724
- JP-A- 2005 278 724
- JP-A- 2007 149 498
- JP-A- 2007 149 498
- US-A1- 2005 177 139
- US-B1- 6 587 482

## Description

### TECHNICAL FIELD

The present invention relates generally to light emission beauty devices and, more particularly, to a light emission beauty device for emitting light emitted from a light source to a biological surface, especially a surface of a biological skin, to treat skin tissue.

### BACKGROUND ART

Previously, a variety of light emission beauty devices have already provided the art for emitting light emitted from a light source to a biological surface, especially a surface of a biological skin, to treat skin tissue with removing hair and inhibiting the development of new hair, etc. One such example is described in Japanese Patent Application Laid-Open No.2005-278724 published in October 13, 2005. This light emission beauty device comprises a tubular body, a light emission device, a lighting circuit and a controller. The light emission device comprises a lens located at a side of one end of the tubular body, and a light source located at the back of the lens. The lighting circuit is configured to activate the light source. The controller is configured to control the lighting circuit. In addition, a xenon tube or a diode, etc. is used as the light source.

Then, the light emission device comprises a touch-sensor comprising three contacts located around an emission opening. The touch-sensor is configured to prohibit the lighting circuit from activating the light source if the touch-sensor doesn't detect contact with the surface of the skin. Therefore, the light emission beauty device can prevent that a light is carelessly emitted to some parts other than the surface of the skin, for example eyes.

By the way, skin's condition varies delicately with ambient temperature or humidity. Also, the touch-sensor is located relatively-near the light source, and thereby the touch-sensor is affected by heat given off from the light source and is easily heated to a high temperature. Thus, even if one brings the touch-sensor into contact with the surface of the skin, the light emission beauty device may not detect contact/noncontact accurately, and may emit unexpected light. Therefore, the light emission beauty device has a problem with lack of adequate safety.

Then, other example is described in Japanese Patent Application Laid-Open No.2004-321401 published in November 18, 2004. The light emission device of this light emission beauty device comprises a spacer at an emission opening. That is, a space is produced between the emission opening and the surface of the skin. Thus, this light emission beauty device can prevent that one suffers a burn caused by the contact of the light emission device heated to a high temperature due to light-emitting with the surface of the skin. However, when the light emission beauty device is subjected to shock by the body's falling and tumble, there is a danger that the light emission beauty device emits unexpected light. Therefore, the light emission beauty device has a problem with lack of adequate safety.

From JP 2005-278724 A a light emission beauty device is known disclosing es sentially all features of the introductory portion of claim 1.

From JP 2007-149498 A a touch-sensor switching device with a touch-sensor for prevention of misoperation is disclosed.

### DISCLOSURE OF THE INVENTION

It is an object of the present invention to provide a light emission beauty device which can prevent unexpected light emission and can improve safety.

A light emission beauty device of the present invention comprises a tubular body, a light emission device, a lighting circuit, a controller, and a light-emitting switch. The light emission device comprises a lens located at a side of one end of the tubular body and a light source located at the back of the lens. The lighting circuit is configured to activate the light source. The controller is configured to control the lighting circuit. The light-emitting switch is configured to supply a light-emitting signal to the controller in accordance with on operation. According to a first aspect of the present invention, the controller is configured to make the lighting circuit activate the light source in response to receiving the light-emitting signal continuously for a predetermined time. In this invention, the controller makes the lighting circuit activate the light source in response to receiving the light-emitting signal continuously for a predetermined time, and thereby even if the light-emitting switch is turned on by the tubular body's falling and tumble or erroneous operation, the light source is not activated. Therefore, the invention can provide a light emission beauty device which can prevent unexpected light emission and can improve safety.

In an embodiment, the controller comprises a counter for counting the number of times of the light source's activation within a predetermined time to obtain a count value. Also, the controller is configured to lengthen time required to activate the light source later in the activation, if the count value reaches a predetermined value. In this invention, the controller lengthens time required to activate the light source later in the activation if the count value reaches a predetermined value, and thereby the light emission beauty device can inhibit rising the temperature of the light emission device with the light source's continuous activation and can prevent damage or erroneous operation.

In an embodiment, the controller comprises a counter for counting the number of times of the light source's activation within a predetermined time to obtain a count value. Also, the controller is configured to prohibit the light source's activation for a given length of time, if the count value reaches a predetermined value. In this invention, the controller prohibit the light source's activation for a given length of time if the count value reaches a predetermined value, and thereby the light emission beauty device can inhibit rising the temperature of the light emission device with the light source's continuous activation and can prevent damage or erroneous operation.

In an embodiment, the lighting circuit comprises a capacitor storing an electric charge for allowing the light source to emit light, and a voltage-detector for detecting voltage value of the capacitor. The controller is configured to determine whether or not the voltage value detected with the voltage-detector just before the light source is activated is in a predetermined range. Also, the controller is configured to prohibit the lighting circuit from activating the light source if the voltage value is out of the predetermined range. In this invention, the controller prohibits the lighting circuit from activating the light source if the voltage value of the capacitor, detected just before the light source is activated, is out of the predetermined range, and thereby even if there is an abnormality in the capacitor due to change of dielectric dissipation factor and thermal runaway, the light emission beauty device can detect the abnormality before the light source is activated. Therefore, the light emission beauty device can prevent that the light source is activated in a state of low-light intensity and low-beauty effect and can prevent damage or erroneous operation.

According to a second aspect of the present invention, the light emission device comprises an emission unit which can be attached to and detached from the end of the tubular body. The tubular body comprises a detection means for detecting whether or not the emission unit is attached to the end of the tubular body. In this invention, the emission unit can be attached to and detached from the end of the tubular body, and thereby the user can easily replace only an old emission unit with a new emission unit upon the end of the emission unit's life and malfunction of the emission unit, and thus cost can be reduced. Also, the tubular body comprises a detection means for detecting whether or not the emission unit is attached to the end of the tubular body, and thereby the light emission beauty device can perform a control corresponding to existence or non-existence of the emission unit.

In an embodiment, the lighting circuit comprises a capacitor storing an electric charge for allowing the light source to emit light and a converter circuit configured to supply an electric power to the capacitor. The converter circuit comprises an electric source, a switching element, and a booster boosting a voltage from the electric source by means of on-off switching of the switching element and supplying the boosted voltage to the capacitor. The tubular body comprises a charging switch configured to supply a charging signal to the controller in accordance with on operation. The controller is configured to make the switching element perform on-off switching in response to the charging signal if the detection means detects the emission unit. Also, the controller is configured to make the switching element maintain off-state even if receiving the charging signal, if the detection means doesn't detect the emission unit. In this invention, the controller makes the switching element maintain off-state even if receiving the charging signal, if the detection means doesn't detect the emission unit, and thereby a current does not flow in the lighting circuit even if the charging switch is accidentally operated on-state, when the emission unit is detached. Then, the invention can prevent electric shock accident and breakdown of the device.

In an embodiment, the controller is configured to allow the lighting circuit to activate the light source in response to the light-emitting signal, if the detection means detects the emission unit. Also, the controller is configured prohibit the lighting circuit from activating the light source even if receiving the light-emitting signal, if the detection means doesn't detect the emission unit. In this invention, the controller prohibits the lighting circuit from activating the light source even if receiving the light-emitting signal, if the detection means doesn't detect the emission unit, and thereby a current does not flow in the lighting circuit even if the lighting switch is accidentally operated on-state, when the emission unit is detached. Then, the invention can prevent electric shock accident and breakdown of the device.

In an embodiment, the lighting circuit comprises a capacitor storing an electric charge for allowing the light source to emit light, a converter circuit configured to supply an electric power to the capacitor, and a current control element. The converter circuit comprises an electric source, a switching element, and a booster boosting a voltage from the electric source by means of on-off switching of the switching element and supplying the boosted voltage to the capacitor. The emission unit comprises a first connector electrically connected to the light source. The tubular body comprises a second connector electrically connected to the capacitor. The capacitor and the light source are electrically connected to each other when the second connector joints the first connector. The current control element is located in an electric path between the capacitor and the second connector and turns the electric path on or off. The controller is configured to make the current control element turn off, if the detection means doesn't detect the emission unit. In this invention, the controller makes the current control element turn off if the detection means doesn't the emission unit, and thereby short-circuit doesn't occur between terminals located in the second connector when the emission unit is detached. Then, the invention can prevent electric shock accident and breakdown of the device more effectively.

According to a third aspect of the present invention, the lighting circuit comprises a capacitor storing an electric charge for allowing the light source to emit light. The controller comprises a counter for counting the number of times of the light source's activation to obtain a count value and a memory for storing a table having different voltage values corresponding to different count values, respectively. The controller is configured to correct a target value to be applied across the capacitor to a voltage value, corresponding to the count value obtained by the counter, in the table. In this invention, the controller corrects to a voltage value, corresponding to the count value obtained by the counter, in the table, and thereby when the light source can not emit sufficient light intensity in a conventional voltage target value due to deterioration, the light emission beauty device can increase the target value and can keep the light intensity constant.

### BRIEF DESCRIPTION OF THE DRAWINGS

Preferred embodiments of the invention will now be described in further details. Other features and advantages of the present invention will become better understood with regard to the following detailed description and accompanying drawings where:
Fig. 1 illustrates a light emission beauty device according to a first embodiment of the present invention, wherein Fig. 1A is a front view and Fig. 1B is a right side view;
Fig. 2A is a cross-sectional view taken along line A-A of Fig. 1A, and Fig. 2B is a cross-sectional view taken along line B-B of Fig. 1B;
Fig. 3 is a diagrammatic perspective view showing the light emission beauty device, without a cover, according to said first embodiment of the present invention;
Fig. 4 illustrates an emission unit of the light emission beauty device according to said first embodiment of the present invention, wherein Fig. 4A is a front view and Fig. 4B is a cross-sectional view taken along line C-C of Fig. 4A;
Fig. 5 is an exploded perspective view showing the emission unit of said first embodiment of the present invention;
Fig. 6 illustrates a cover of the light emission beauty device according to said first embodiment of the present invention, wherein Fig. 6A is a front view and Fig. 6B is a cross-sectional view taken along line D-D of Fig. 6A;
Fig. 7 is a circuit configuration diagram of the light emission beauty device according to said first embodiment of the present invention; and
Fig.8 is a flowchart for explaining an operation of the light emission beauty device according to said first embodiment of the present invention.

### BEST MODE FOR CARRYING OUT THE INVENTION

### (First Embodiment)

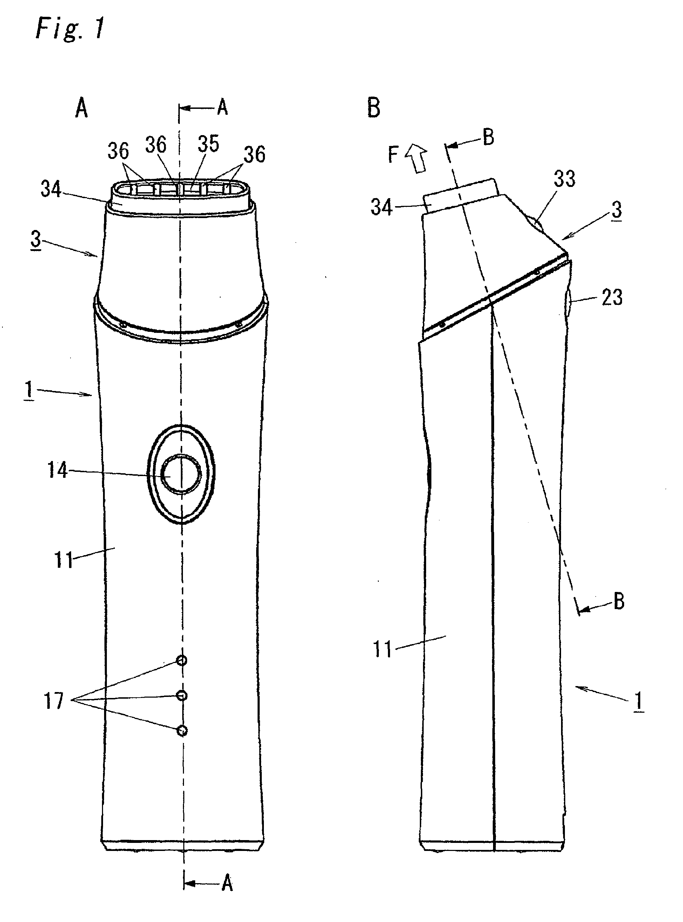

Hereinafter, a first embodiment of the present invention is explained with reference to Figs. 1 to 8.

A light emission beauty device 1 of the present embodiment emits light to a biological surface, especially a surface of a biological skin, to treat skin tissue with removing hair and inhibiting the development of new hair, etc.

Then, the light emission beauty device 1, as shown in Figs. 1 to 3, comprises a tubular body 11 which can be grasped in one hand by a user, a cover 3, an emission unit 4, a lighting circuit 5, a controller 6, and a light-emitting switch 7. As specific explanation will hereinafter be described, the emission unit 4 of the present embodiment can be attached to and detached from the tubular body 11.

The tubular body 11, as shown in Figs. 1 to 3, is formed into a tube shape so that its longitudinal direction extends in a vertical direction, and the emission unit 4 is held in an upper end of the tubular body 11. In addition, hereinafter, the upper end of the tubular body 11 is called an emission end 2.

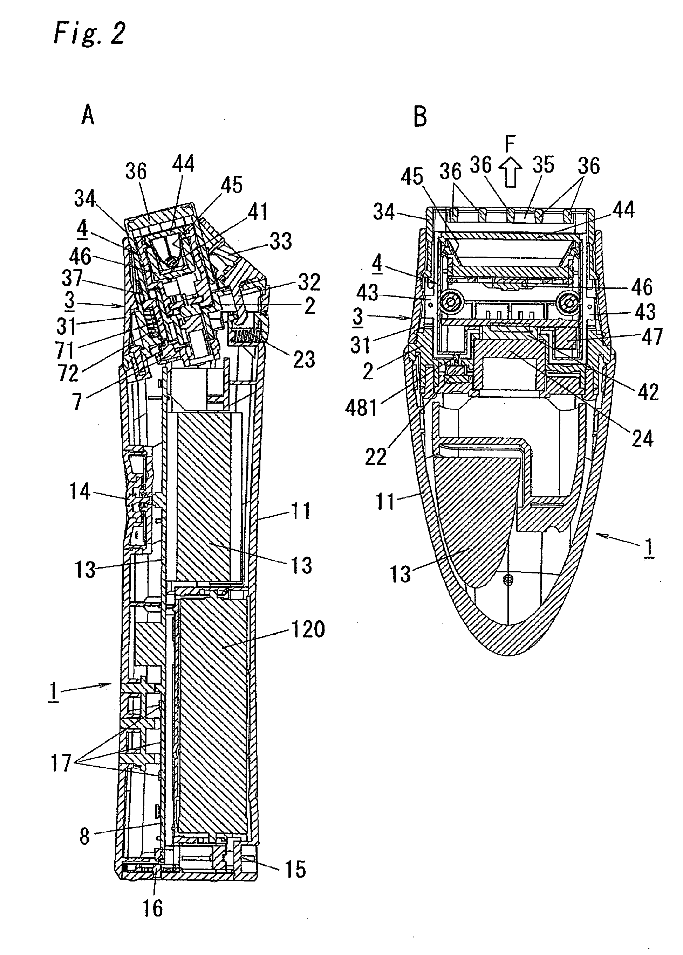

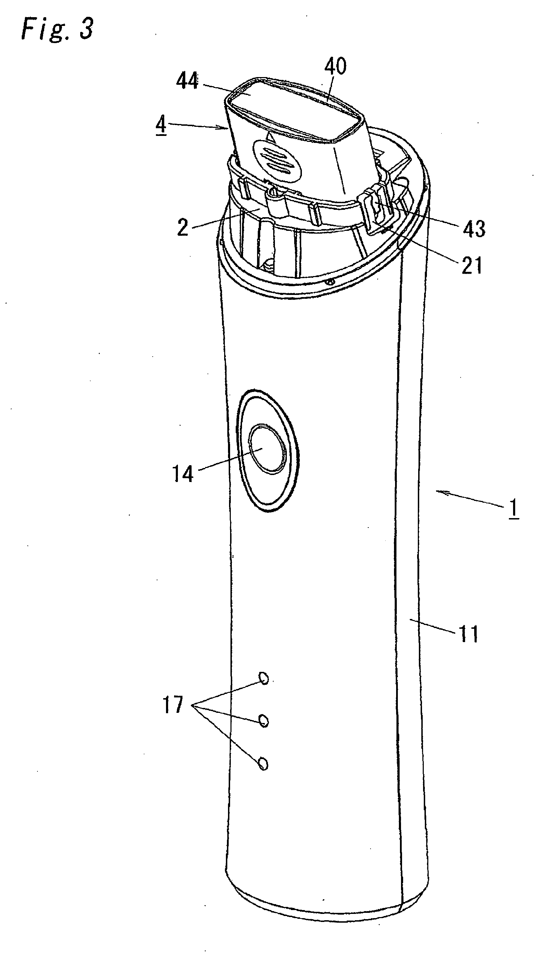

As shown in Figs. 2 and 3, a pair of attaching sections 21, 21, a detection switch 22, a catch section 23, and a second connector 24 are located in the emission end 2. The attaching sections 21, 21, as shown in Fig. 3, are formed into generally a C-shape and projects from right and left ends of the emission end 2, respectively. As shown in Fig. 2B, the detection switch 22 is located, so as to be exposed, in the left side of the emission end 2, and is a detection means for detecting the emission unit 4. Specifically, when the emission unit 4 is attached to the emission end 2, the detection switch 22 is pushed by a projection portion 481 of the emission unit 4 described later, and thereby is operated on-state. Then, the detection switch 22 outputs a detection signal to the controller 6. The back of the emission end 2, as shown in Fig. 2A, has a downwardly-recessed portion, and thereby the catch section 23 is formed. The second connector 24 projects upwardly from generally a center of the emission end 2.

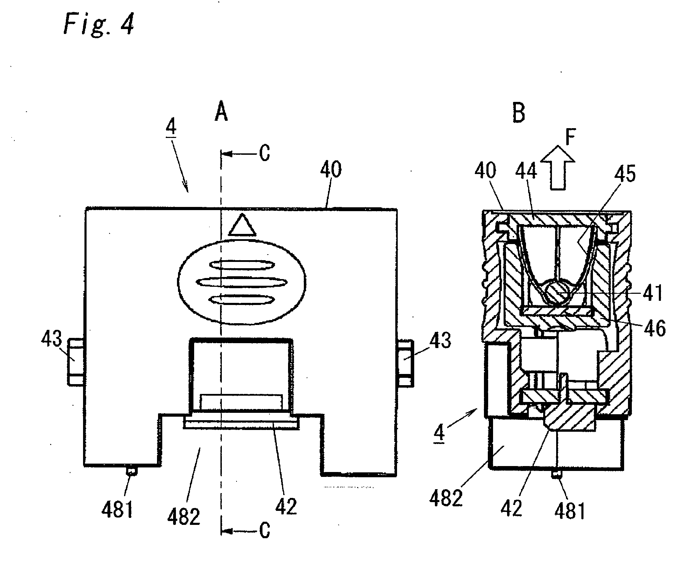

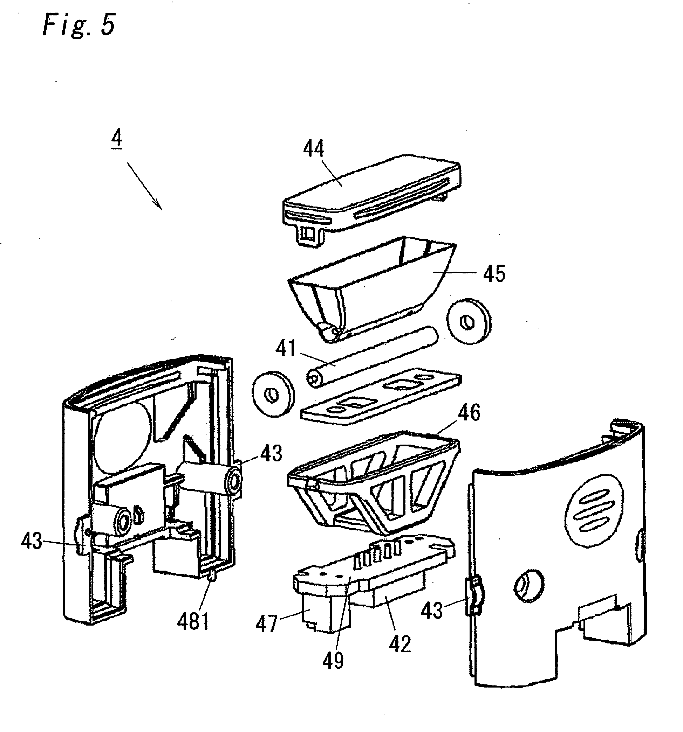

The emission unit 4, as shown in Figs. 4 and 5, is formed into a shape of a rectangular cylinder, and is a light emission device which has an emission opening 40 opened into generally a rectangle shape at its upper side. Here, in the emission unit 4, a lens 44 is located so as to cover the emission opening 40. A base unit 46, formed into a C-shape in cross-section, is located at the back of the lens 44 within the emission unit 4, as shown in Fig. 4B. Then, a cup-shaped reflector 45, comprising a reflection surface inside, is held within the emission unit 4 via the base unit 46. In addition, the reflector 45 is configured so that an outer diameter of its upper end's periphery is generally equal to a transverse size of the emission opening 40.

Alight source 41 is located at an inside bottom part of the reflector 45. Light emitted from the light source 41 is efficiently emitted from the emission opening 40 through the reflector 45 and the lens 44. For example, a tubular-ball-shaped xenon tube or a diode is used as the light source 41. In addition, hereinafter, a direction of light emitted from the emission unit 4 is called a light emission direction F, as shown in Fig. 4B.

As shown in Figs. 3 and 4A, attachment projections 43, 43 are projected at the bottoms of right and left side surfaces of the emission unit 4, respectively. An attachment projection 43 engages with and disengages from the attaching section 21 of the emission end 2, as shown in Fig. 3, and thereby the emission unit 4 can be attached to and detached from the emission end 2. Therefore, the user can easily replace only an old emission unit 4 with a new emission unit 4 upon the end of the emission unit 4's life and malfunction of the emission unit 4, and thus cost can be reduced.

As shown in Figs. 4 and 5, the projection portion 481 is projected downwardly at the left end of the outside bottom of the emission unit 4. Then, a recess 482 is formed so as to sag in the light emission direction F, in the center of the outside bottom of the emission unit 4. A base plate 49 is housed in the bottom within the emission unit 4. Then, a first connector 42, to which the second connector 24 described above is attached, is electrically connected to the light source 41 and is mounted on the base plate 49 so as to be exposed from the recess 482. In addition, the base plate 49 carries a trigger transformer 47 supplying a trigger voltage to the light source 41, and various circuit components constituting a unit circuit section (not shown) for connecting the first connector 42 and the light source 41 electrically, as shown in Fig. 5.

As shown in Figs. 1, 2A and 6, the cover 3 comprises a cover body 31, a float block 34 having a light-blocking effect, a latch section 32, and an extended part 37. An upper end of the cover body 31 is opened into generally a rectangular shape, and a lower end of the cover body 31 is opened into generally an elliptical shape, and then the cover body 31 is formed so as to expand from the upper end toward the lower end. In addition, an outline of the lower end's periphery of the cover body 31 is set to be generally equal to that of the emission end 2.

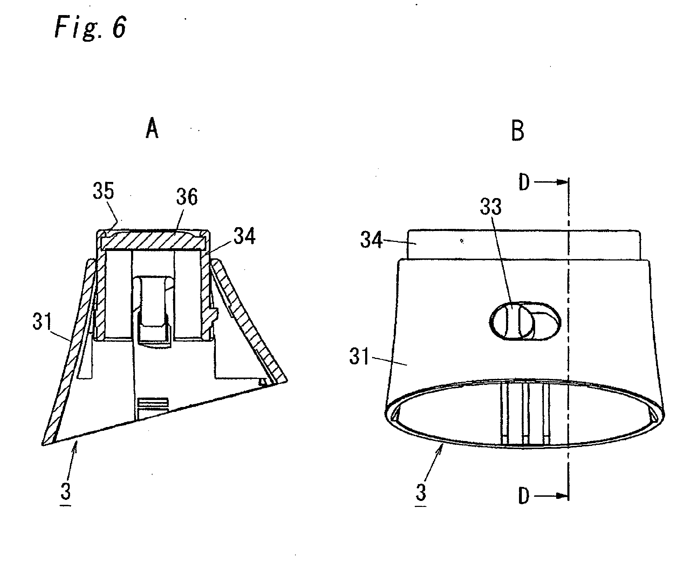

The float block 34 is formed into generally a rectangular cylinder shape, and its both ends is opened, and an upper end of the both ends is an injection hole 35. As shown in Fig. 1A, a plurality of translucent gratings 36 are embedded in the injection hole 35. Then, the float block 34 is floatably held in a position of the upper end of the cover body 31 while being biased in the light emission direction F by an elastic member (not shown). However, a float lock section 33 is located in the cover body 31, as shown in Fig. 6B, and fixes the float block 34 to the cover body 31, and thereby prohibits the float block 34 from moving floatably, or releases the prohibition.

The latch section 32 is extended downwardly from the outside bottom of the cover body 31, and its end is formed into a hook shape, as shown in Fig. 2A. Then, the latch section 32 can engage with the catch section 23 of the emission end 2 disengageably, and thereby the cover 3 is attached to the tubular body 11. At this time, the float block 34 covers the outside of the emission unit 4, as shown in Figs. 1 and 2. In addition, the emission opening 40 of the emission unit 4 is located so as to face the injection hole 35 within the cover 3. That is, light emitted form the emission opening 40 is emitted toward the outside of the cover 3 through the injection hole 35.

The extended part 37 is formed so as to be extended from an opening lower end's periphery of the float block 34 in the opposite direction of the light emission direction F, as shown in Fig. 2A. Then, the extended part 37 pushes the light-emitting switch 7 located in the emission end 2's side, when the float block 34 moves in the opposite direction of the light emission direction F. In addition, a composition of the light-emitting switch 7 and an operation of the light-emitting switch 7 with the extended part 37 are described later in detail.

Thus, in the light emission beauty device 1, the biological surface does not contact with the emission unit 4, even if the light source 41 emits light while the injection hole 35 is pressed against the biological surface. That is, the light emission beauty device 1 prevents a user from contacting with the emission unit 4 heated to a high temperature due to light-emitting of the light source 41 and from suffering burns. Thus, the light emission beauty device 1 has high safety. In addition, the float block 34 has a light-blocking effect, and thereby light emitted from the light source 41 does not escape from other than the injection hole 35. Therefore, the light emission beauty device 1 can emit light to the biological surface efficiently. Moreover, even if the float block 34 is pressed against the biological surface in the light emission direction F while the injection hole 35 faces the biological surface, the float block 34 moves in the opposite direction of the light emission direction F. Thus, the light emission beauty device 1 can attenuate an impact given to the biological surface. At this time, a space is not produced between the biological surface and the float block 34 by means of biasing of the above-mentioned elastic member, and light does not escape in a direction other than the light emission direction F.

The light-emitting switch 7 is configured to supply a light-emitting signal to the controller 6 in accordance with on operation, and is located adjacent to the emission end 2 within the tubular body 11, as shown in Fig. 2A. Specifically, a hole is drilled toward the light-emitting switch 7 in the emission end 2. As shown in Fig. 2A, a receiving part 71, formed into generally an L-shape in cross-section, and an elastic body 72, biasing the receiving part 71 in the light emission direction F, are located in the hole. For example, the elastic body 72 is a spring. Then, when the emission unit 4 and the cover 3 are attached to the tubular body 11, the extended part 37 is located so as to face the receiving part 71.

Here, when the float block 34 is pressed against the biological surface, the float block 34 and the extended part 37 move toward the emission end 2's side while resisting a biasing force of the elastic member of the cover body 31. Then, the extended part 37 presses the receiving part 71 while resisting a biasing force of the elastic body 72, and thereby the light-emitting switch 7 is operated on-state. At this time, the light-emitting switch 7 supplies the light-emitting signal to the controller 6. In addition, when the float block 34 is separated from the biological surface, the float block 34 floats in the light emission direction F due to the biasing force of the elastic member of the cover body 31. Then, on-state of the light-emitting switch 7 is released, and the light-emitting switch 7 stops supplying the light-emitting signal to the controller 6.

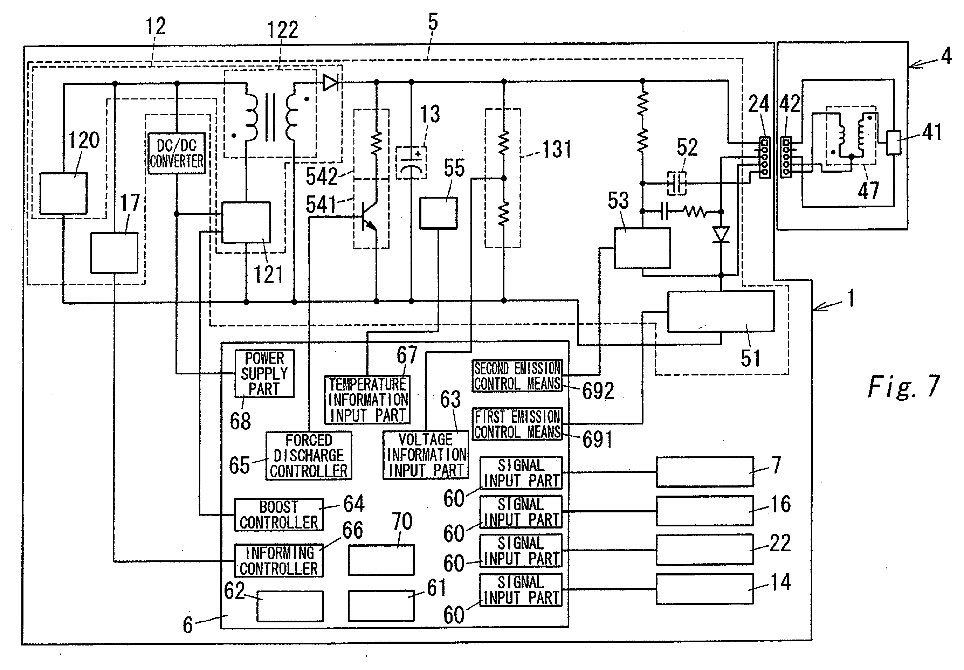

The lighting circuit 5 is configured to activate the light source 41, and comprises a converter circuit 12, a capacitor 13, current control elements 51, 53, a temperature-detector 55, a voltage-detector 131, a forced discharge section 542, a forced discharge control element 541, and an alarm 17, as shown in Fig. 7. The capacitor 13 stores an electric charge for allowing the light source 41 to emit light, and is electrically connected to the second connector 24. The temperature-detector 55 comprises a temperature sensor such as a thermistor, and detects a temperature of the capacitor 13. The voltage-detector 131 comprises two resistors, and detects a stored voltage of the capacitor 13. The forced discharge section 542 forcibly discharges the electric charge stored in the capacitor 13. The forced discharge control element 541 is controlled by the controller 6 to turn an electric path of the forced discharge section 542 on or off. The alarm 17 comprises, for example, a light-emitting diode or a buzzer, and informs an external about a state of light emission and existence or non-existence of abnormality.

The converter circuit 12 is configured to supply an electric power to the capacitor 13, and comprises an internal electric source 120, a switching element 121, and a booster 122, as shown Fig. 7. Here, the booster 122 boosts a voltage from the internal electric source 120 by means of on-off switching of the switching element 121 and supplies the boosted voltage to the capacitor 13. The internal electric source 120 is, for example, a battery.

The current control elements 51, 53 are located in an electric path between a negative electrode of the capacitor 13 and the second connector 24, and turn the electric path on or off, as shown in Fig. 7. Specifically, the output of the current control element 51 and the input of the current control element 53 are connected to each other, as shown in Fig. 7, and then a junction between the elements 51, 53 is electrically connected to a midway between two inductors of the trigger transformer 47 through the first and second connectors 42, 24. In addition, transistors are used in the current control elements 51, 53. In particular, it's preferred that Insulated Gate Bipolar Transistor (IGBT) is used in the current control element 51.

Then, a circuit substrate 8, carrying various circuit components constituting the lighting circuit 5, is housed within the tubular body 11, as shown in Fig. 2A.

The tubular body 11 of the present embodiment is provided in the center of its front with a charging switch 14, which is configured to make the capacitor 13 start storing the electric charge in accordance with on operation of a user, as shown in Fig. 1A. The charging switch 14 is a type of switch, which can project from and retract into, and then a projected situation of the switch 14 shows off-state and a retracted situation shows on-state. Then, if the user continues to press the charging switch 14 with the retracted situation (that is, on-state is kept), the electric charge is stored in the capacitor 13. In addition, the charging switch 14 is set to flash with or sag to be lower than the front of the tubular body 11, upon off-state. Thus, the light emission beauty device 1 can prevent erroneous operation that the charging switch 14 turns on, etc. upon falling and tumble of the tubular body 11.

In addition, an automatic-return type of a momentary action switch is used in the charging switch 14 and the above-mentioned other switch, and turns on only upon pressing. However, an alternate action switch may be used in the charging switch 14 and the above-mentioned other switch. Then, the light emission beauty device 1 may be configured so that light-emitting of the light source 41 is prohibited or the charging switch 14 is automatically turned off, if the charging switch 14 is not operated off-state after the charge of the capacitor 13 is completed.

In addition, the tubular body 11 is provided in the lower end of its back with a charging terminal 15, which is electrically connected to the internal electric source 120, as shown in Fig. 2A. Then, for example, a charging means (not shown), connected to a commercial power source, is connected to the charging terminal 15, and thereby the internal electric source 120 is charged.

The controller 6 is a microcomputer and controls various switching elements of the lighting circuit 5 in accordance with signals received from various switches. Then, as shown in Fig. 7, the controller 6 comprises a plurality of signal input parts 60, a counter 61, a memory 62, a voltage information input part 63, a boost controller 64, a forced discharge controller 65, an informing controller 66, a temperature information input part 67, a power supply part 68, a first emission control means 691, a second emission control means 692, and a time measurement part 70.

As shown in Fig. 7, the plurality of signal input parts 60 are electrically connected to the charging switch 14, the light-emitting switch 7, the detection switch 22, and a reset switch 16 described later, respectively, and receives signals from various switches.

The counter 61 counts the number of times of the light source 41's activation within a predetermined time (for example, within one hour) to obtain a count value. In addition, the number of times of the light source 41's activation means the number of times the light source 41 normally stops emitting light.

The memory 62 stores computational decisions and the count value, and further stores an accumulative count value accumulated from time when the light source 41 starts the operation for the first time until now, and a table having different voltage values corresponding to different count values or ranges, respectively. In addition, it's preferred that the memory 62 is a nonvolatile memory element, which can store without an electric power supply.

The boost controller 64 is configured to control on-off switching of the switching element 121 in response to receiving the charging signal from the charging switch 14 and the detection signal from the detection switch 22.

The voltage information input part 63 is electrically connected to the voltage-detector 131, and receives an electric signal showing a voltage of the capacitor 13 detected by the voltage-detector 131, and performs A/D conversion of the electric signal, as shown in Fig. 7.

The temperature information input part 67 is electrically connected to the temperature-detector 55, and receives an electric signal showing a temperature of the capacitor 13 detected by the temperature-detector 55, and performs A/D conversion of the electric signal, as shown in Fig. 7.

The forced discharge controller 65, as explained above, makes the forced discharge control element 541 turn on and makes the forced discharge section 542 discharge the charged voltage of the capacitor 13 forcibly, when determining that there is an abnormality in the lighting circuit 5. At the same time, the informing controller 66 makes the alarm 17 inform an external about occurrence of abnormality. In addition, the forced discharge controller 65 makes the forced discharge control element 541 turn off again, when the voltage of the capacitor 13 decreases to an initial voltage of pre-charge.

The power supply part 68 is located in order to drive the above-mentioned parts of the controller 6, and is supplied an electric power by the internal electric source 120 through a DC/DC converter, as shown in Fig. 7.

The first emission control means 691 makes the current control element 51 turn on, in response to the light-emitting signal received from the light-emitting switch 7. The second emission control means 692 makes the current control element 53 turn on, in response to a time when the light-emitting signal is continuously received. The time measurement part 70 is configured to measure a time spent on the charge of the capacitor 13.

In addition, the reset switch 16 is located at the other end of the emission end 2 of the tubular body 11, as shown in Fig. 2. The reset switch 16 is configured to supply a reset signal to the controller 6 in accordance with on operation. The controller 6 resets, for example, the accumulative count value stored in the memory 62 to zero when receiving the reset signal. Here, the reset switch 16 does not operate automatically in accordance with attachment and detachment of the emission unit 4, but the user presses to operate the reset switch 16, upon an emergency stop due to the tubular body 11's falling or erroneous operation, or upon replacement of the emission unit 4. Accordingly, the present device 1 can prevent that the count value is reset to zero not upon the replacement of the emission unit 4 but upon checking and cleaning and thereby the end of the emission unit 4's life is not determined accurately. Furthermore, the reset switch 16 is configured to be able to reset only during a period from attachment and detachment of the emission unit 4 to operation of the light source 41. Thus, the present device 1 can prevent the reset switch 16 from resetting in a state without attachment and detachment of the emission unit 4.

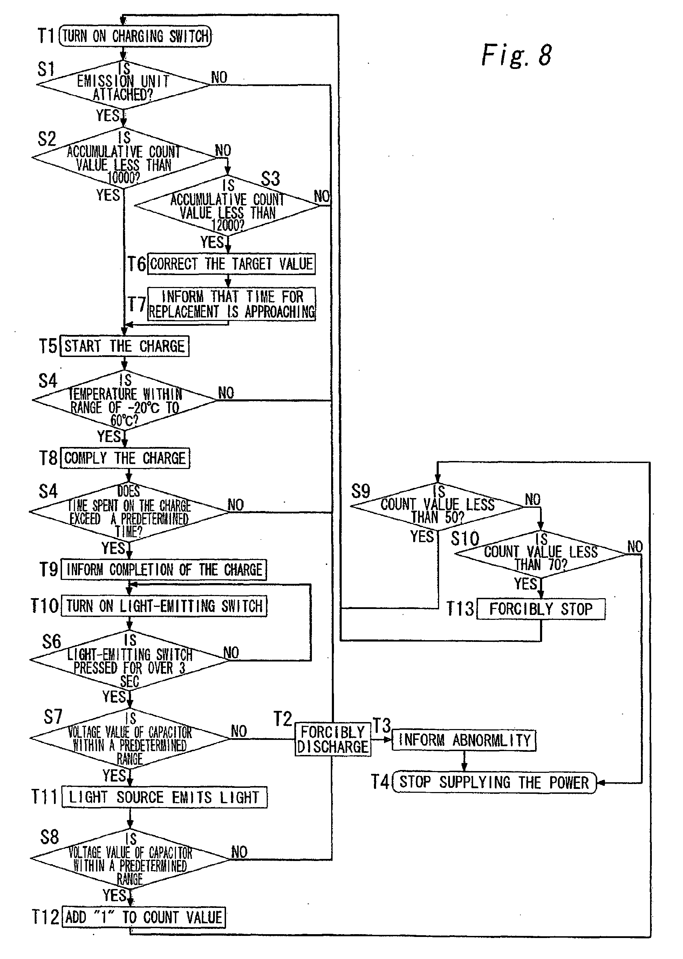

Hereinafter, an operation of the light emission beauty device 1 of the present embodiment is explained with reference to a flowchart of Fig. 8.

First, as shown in Fig. 8, a user operates the charging switch 14 on-state by means of pressing (T1), and then the above-mentioned DC/DC converter is operated on-state, and the present device 1 starts to supply a electric power to the power supply part 68 of the controller 6. Then, the charging switch 14 supplies a charging signal to the controller 6 in accordance with said operation. Here, the controller 6 determines whether or not a detection signal is received from the detection switch 22 when receiving the charging signal. That is, as shown in Fig. 8, the controller 6 determines whether or not the emission unit 4 is attached to the tubular body 11 (S1). Then, if the emission unit 4 is attached to the tubular body 11, the processing moves to S2. However, even if the controller 6 receives the charging signal, the processing does not move to S2 if the emission unit 4 is not attached to the tubular body 11. Instead, as shown in Fig. 8, the force discharge section 542 forcibly discharges a voltage charged previously in the capacitor 13 (T2). In addition, the controller 6 makes the alarm 17 inform an external about occurrence of abnormality (T3), and stops to supply the electric power to the power supply part 68 (T4).

In addition, even in the middle of charging the capacitor 13, the controller 6 immediately makes the switching element 121 turn off to stop the charge when the detection signal from the detection switch 22 is interrupted. Therefore, even if the charging switch 14 is accidentally operated on-state in a state where the emission unit 4 is detached, an electric current does not flow to the lighting circuit 5, and then the light emission beauty device 1 can prevent electric shock accident and breakdown of the device.

Next, the controller 6 references the accumulative count value, accumulated from time when the reset switch 16 is pressed until now, stored in the memory 62 to determine whether or not the accumulative count value reaches 10000 (S2), as shown in Fig. 8. If the accumulative count value is less than 10000, the controller 6 determines that the light source 41 can emit sufficient light intensity, and starts the charge of the capacitor 13 (T5). That is, the controller 6 supplies a driving signal to the switching element 121. The switching element 121 receives the driving signal and performs on-off action (on-off switching), and thereby the booster 122 boosts a voltage from the internal electric source 120, and then the capacitor 13 is charged.

On the other hand, if the count accumulative value reaches 10000, the controller 6 further determines whether or not the accumulative count value reaches 12000 (S3), as shown in Fig. 8. If the accumulative count value is lees than 12000, the controller 6 determines that the light source 41 can not emit sufficient light intensity in a conventional voltage target value supplied to the capacitor 13. So the controller 6 of the present embodiment corrects the voltage target value supplied to the capacitor 13 (T6). That is, the controller 6 references the table stored in the memory 62 and obtains a voltage value, corresponding to the accumulative count value, in the table. Then, the controller 6 controls the switching element 121 so as to correct the voltage target value, supplied to the capacitor 13, to the voltage value, corresponding to the count value, and starts the charge of the capacitor 13 (T5). In addition, the controller 6 makes the alarm 17 inform an external that time for replacement of the emission unit 4 is approaching, through the informing controller 66 (T7). Therefore, even if the light source 41 can not emit sufficient light intensity in a conventional voltage target value due to deterioration, the light emission beauty device 1 can increase the target value and can keep the light intensity constant.

Furthermore, if the accumulative count value reaches 12000, the controller 6 determines that the light source 41 completely becomes deteriorated. Then, the controller 6 discards the received charging signal, and makes the switching element 121 turn off, and then prohibits the charge of the capacitor 13. In addition, the controller 6 forcibly discharges a voltage charged previously in the capacitor 13 through the forced discharge section 542 (T2), and then makes the alarm 17 inform an external that it is necessary to replace due to the end of the emission unit 4's life, through the informing controller 66 (T3), and then stops to supply the electric power to the power supply part 68 (T4). In addition, thereafter, the controller 6 only stops to supply the electric power to the power supply part 68 and keeps to prohibit the charge of the capacitor 13, even if receiving the charging signal from the charging switch 14. Then, the controller 6 only makes the alarm 17 inform for a given length of time, and then automatically stops to supply the electric power to the power supply part 68. This state is continued, until a user replaces the old emission unit 4 with the new emission unit 4 and the accumulative count value in the memory 62 is reset to zero by means of the rest switch 16.

The controller 6 determines whether or not a temperature of the capacitor 13, detected by the temperature-detector 55 through the temperature information input part 67, is within range of a predetermined temperature (for example, minus 20 degrees C to 60 degrees C) stored previously in the memory 62, while charging the capacitor 13 (S4), as shown Fig. 8. Then, if the temperature of the capacitor 13 is not within range of the predetermined temperature, the controller 6 determines that there is an abnormality and prohibits the light source 41's activation. Specifically, the controller 6 makes the switching element 121 maintain off-state through the boost controller 64, and stops charging the capacitor 13. The controller 6 forcibly discharges a voltage charged previously in the capacitor 13 through the forced discharge section 542 (T2), and makes the alarm 17 inform an external about occurrence of abnormality (T3), and then stops to supply the electric power to the power supply part 68 (T4). Further, the controller 6 prohibits an operation for recharging the capacitor 13. In addition, the controller 6 determines whether or not the temperature is within range, only while charging the capacitor 13, in Fig. 8. However, the controller 6 may also does it while not charging the capacitor 13.

After the charge of the capacitor 13 is completed (T8), the controller 6 determines whether or not a time, spent on the charge of the capacitor 13, measured by the time measurement part 70 exceeds a predetermined time stored in the memory 62 (S5). Then, if the time spent on the charge of the capacitor 13 exceeds the predetermined time, the controller 6 determines that the charge was normally completed and makes the alarm 17 inform an external about completion of the charge (T9). On the other hand, if the time spent on the charge of the capacitor 13 does not exceed the predetermined time, the controller 6 determines that the charge was not normally completed and forcibly discharges a voltage charged in the capacitor 13 through the forced discharge section 542 (T2). Then, the controller 6 makes the alarm 17 inform an external about occurrence of abnormality (T3), and then stops to supply the electric power to the power supply part 68 (T4).

Then, when the light emission beauty device 1 is pressed against the biological surface after completion of the charge, the operation of the device 1 is specifically described below.

First, a user exposes the injection hole 35 to a region of the biological surface, to which the user wants to emit light for beauty effect, and presses the float block 34 against the region, and turns on the light-emitting switch 7 (T10). At this time, the float block 34 moves in the opposite direction of the light emission direction F. Then, the light-emitting switch 7 supplies the light-emitting signal to the controller 6. However, when the float block 34 is fixed by the float lock section 33, the float block 34 does not move in the opposite direction even if the user presses the float block 34 against the biological surface.

As shown in Fig. 7, the controller 6 makes the current control element 51 turn on through the first emission control means 691 and charges a trigger capacitor 52 for a trigger voltage, when receiving the light-emitting signal. Here, the controller 6 of the present embodiment makes the current control element 53 turn on through the second emission control means 692 and supplies an electric current to the trigger transformer 47, if continuously detecting the light-emitting signal for a predetermined time (for example, 3 to 5 sec) stored in the memory 62. That is, the controller 6 determines whether or not the light-emitting switch 7 is held to be pressed for at least over 3 sec (S6), as shown in Fig. 8. If the light-emitting switch 7 is held to be pressed for over 3 sec, the controller 6 determines that the light-emitting switch 7 was operated accurately by the user. Then, the processing moves to next S7. However, if the light-emitting switch 7 is not held to be pressed for over 3 sec, the controller 6 determines that the light-emitting switch 7 was pressed due to the tubular body 11's falling and tumble or erroneous operation. Then, the processing returns to a state before the light-emitting switch 7 is turned on.

Then, although not shown in Fig. 8, the controller 6 of the present embodiment prohibits the light source 41's activation, if the detection switch 22 does not detect the emission unit 4 when receiving the light-emitting signal. Therefore, an electric current does not flow to the lighting circuit 5, even if the light-emitting switch 7 is accidentally operated in a state that the emission unit 4 is detached, and thus the light emission beauty device 1 can prevent electric shock accident and breakdown of the device.

Next, when the trigger voltage, boosted in the trigger transformer 47 by means of on switching of the current control element 53, is supplied to the light source 41, an inside of the light source 41 is ionized and becomes a light-emissionable state. Then, an electric charge, charged in the capacitor 13, is discharged in the ionized inside of the light sources 41, and the light source 41 emits light (T11). Here, a distribution of light intensity emitted from the light source 41 is uniformized through the reflector 45 and the lens 44. Then, the light is emitted to the biological surface through the emission opening 40 and the injection hole 35.

In addition, after the charge of the capacitor 13, when a user does not press the float block 34 against the biological surface for a long time due to getting a visitor and having a phone call, etc. and there is an abnormality in the capacitor 13, the voltage value of the capacitor 13 may be out of a predetermined range. On the other hand, the controller 6 of the present embodiment determines whether or not a voltage value, detected by the voltage-detector 131 through the voltage information input part 63 just before the light source 41's activation, is in a predetermined range stored previously in the memory 62 (S7), as shown in Fig. 8. The above-mentioned "just before the light source 41's activation" means a time when the trigger capacitor 52 is being charged, or a time when the current control element 53 is turned on. Then, if the voltage value detected by the voltage-detector 131 is out of the predetermined range, the controller 6 determines that an abnormity occurred and prohibits the light source 41's activation and forcibly discharges a voltage charged previously in the capacitor 13 through the forced discharge section 542 (T2). Then, the controller 6 makes the alarm 17 inform an external about occurrence of abnormality (T3), and then stops to supply the electric power to the power supply part 68 (T4). Thus, even if abnormality occurs in the capacitor 13 due to change of dielectric dissipation factor and thermal runaway, etc, the light emission beauty device 1 can detect the abnormality before the light source 41 is activated. Then, the light emission beauty device 1 can prevent that the light source 41 is activated in a state of low-light intensity and low-beauty effect, and can prevent damage or erroneous operation.

Further, after completion of the light-emitting, the controller 6 immediately makes the current control element 51, 53 turn off, and determines whether or not a voltage value of the capacitor 13, measured through the voltage-detector 131, is in a predetermined range (S8). If a voltage value after completion of the light-emitting is out of the predetermined range, the controller 6 determines that an abnormity occurred and prohibits the next charge of the capacitor 13. Then, the controller 6 forcibly discharges a voltage charged in the capacitor 13 through the forced discharge section 542 (T2). Then, the controller 6 makes the alarm 17 inform an external about occurrence of abnormality (T3), and then stops to supply the electric power to the power supply part 68 (T4).

If the voltage value of the capacitor 13 after completion of the light-emitting is in the predetermined range, the controller 6 determines that the light source 41 was normally activated. Then, the counter 61 adds "1" to a count value counted for a predetermined time (for example, for one hour) (T12), as shown in Fig. 8. Furthermore, the memory 62 stores the accumulative count value, which is the number of times of the light source's activation from a previous time when the reset switch 16 was pressed until now. Thus the counter 61 also adds "1" to the accumulative count value. In addition, in regard to addition of the number of times of the activation, other than the above-mentioned method, a photodiode may be located between the reflector 45 and lens 44 or in the emission opening 40 or in the injection hole 35. Then, every time the photodiode detects light-emitting, the counter 61 may add "1" to the count value.

Then, the controller 6 determines whether or not a count value counted for a predetermined time reaches a predetermined value (S9 and S10), as shown in Fig. 8. Specifically, the memory 61 stores two predetermined values (for example, "50" and "70"). First, the controller 6 compares the count value with each of two predetermined values. If the count value is less than "50", the controller 6 controls without prohibiting the light source 41's activation later, and then the processing returns to a state before the charging switch 14 is turned on.

If the count value is equal to or more than "50" but less than "70", the controller 6 determines that a heat release of the emission unit 4 can not be fulfilled due to a continuous activation of the light source 41 and thereby a temperature of the emission unit 4 keeps increasing. Then, the controller 6 lengthens time required to activate the light source later in the activation. Specifically, the controller 6 forcibly stops the activation for a while (T13), and then the processing returns to a state before the charging switch 14 is turned on, as shown in Fig. 8. In addition, even if the charging switch 14 and the light-emitting switch 7 are operated on-state while the activation is forcibly stopped, the controller 6 discards the various signals transmitted from them.

If the count value is equal to or more than "70", the controller 6 determines that a temperature of the emission unit 4 increases to near a fatigue limit value due to a heat associated with light-emitting and the device 1 has a risk of breakdown caused by heat deformation and the user has risk of a burn. Then, the controller 6 stops to supply the electric power to the power supply part 68 (T4). Then, the controller 6 prohibits the light source 41's activation for a given length of time. Specifically, even if the charging switch 14 and the light-emitting switch 7 are operated on-state and the controller 6 receives signals from the switch 14 and 7, the controller 6 discards their signals and prohibits the capacitor 13 from charging and prohibits the light source 41 from emitting light for a given length of time. Then, the controller 6 makes the alarm 17 inform an external that the device 1 is dangerous due to high temperature. Then, the controller 6 controls so as to promote a heat loss of the emission unit 4 in order to decrease the temperature of the emission unit 4 for the given length time. As explained above, the controller 6 stops the activation and prohibit light-emitting in response to the count value counted for a predetermined time. Therefore, the light emission beauty device 1 can inhibit rising the temperature of the emission unit 4 with the light source 41's continuous activation, and can prevent damage or erroneous operation.

As explained above, the light emission beauty device 1 of the present embodiment comprises the light-emitting switch 7 which is configured to supply the light-emitting signal to the controller 6 in accordance with on operation. Then, the controller 6 makes the lighting circuit 5 activate the light source 41 in response to receiving the light-emitting signal continuously for a predetermined time. That is, even if the light-emitting switch 7 is turned on by the tubular body 11's falling and tumble or erroneous operation, the light source 41 is not activated. Therefore, the light emission beauty device 1 can prevent unexpected light emission and can improve safety.

By the way, in the lighting circuit 5 of the present embodiment, two current control elements 51, 53 are located between the negative electrode of the capacitor 13 and the second connector 24, as explained above. Then, the controller 6 makes the first and second emission control means 691, 692 turn off the two current control elements 51, 53, if not receiving the detection signal from the detection switch 22. That is, when the emission unit 4 is detached from the tubular body 11, the second connector 24 is not electrically connected to the capacitor 13. Thus, short-circuit doesn't occur between terminals of the second connector 24, and the light emission beauty device 1 can prevent electric shock accident and breakdown of the device more effectively.

In addition, numerical values of the above-mentioned predetermined value and predetermined temperature range can be changed suitably and are not limited.

### (Second Embodiment)

Hereinafter, a light emission beauty device 1 of the present embodiment is explained. In addition, the same composition elements as the first embodiment are putted the same numerals on, in order to make the present embodiment clear.

In the light emission beauty device 1 of the first embodiment, the user presses the reset switch 16 after an emergency stop due to the tubular body 11's falling or erroneous operation, or after replacement of the emission unit 4, and thereby the accumulative count value stored in the memory 62 is reset to zero.

On the other hand, the controller 6 of the present embodiment has a feature in a point of operating as follows. That is, when the accumulative count value stored in the memory 62 reaches a predetermined value and the detection switch 22 is turned off and then is turned on again, the controller 6 determines that the user replaced the old emission unit 4 with the new emission unit 4. Then, the controller 6 automatically resets the accumulative count value stored in the memory 62.

Therefore, if the accumulative count value is less than a predetermined value, the accumulative count value is not reset even if the emission unit 4 is detached upon checking and cleaning. Then, the user does not have to go to the trouble of operating the reset switch 16 upon replacement of the emission unit 4, and workability can be improved.

### (Third Embodiment)

Hereinafter, a light emission beauty device 1 of the present embodiment is explained. In addition, the same composition elements as the first embodiment are putted the same numerals on, in order to make the present embodiment clear.

The light emission beauty device 1 of the present embodiment has a feature in a point of comprising a memory (not shown), which stores a count value obtained by counting the number of times of the light source 41's activation, located in the emission unit 4's side, other than the memory 62 of the controller 6 of the first embodiment.

The controller 6 of the present embodiment acquires the count value stored in the memory located in the emission unit 4's side, whenever receiving the detection signal from the detection switch 22. Then, the controller 6 rests the count value stored in the memory 62 to zero, or overwrites the count value stored in the memory 62 at the count value stored in the memory located in the emission unit 4. Herewith, when the user replaces the emission unit 4 and attaches the in-use emission unit 4 to the other tubular body 11 due to breakdown of the tubular body 11, the user does not have to go to the trouble of operating the reset switch 16. Furthermore, the light emission beauty device 1 can updates the number of times of the light source 41's activation to an appropriate value. Therefore, the light emission beauty device 1 can prevent that a difference is produced between the count value stored in the memory 62 and the number of times of the light source 41's activation due to forgetting to press the reset switch 16 and mischief, and then can improve safety.

Although the present invention has been described with reference to certain preferred embodiments, numerous modifications and variations can be made by those skilled in the art without departing from the scope of this invention as defined by the claims.

## Claims

1. A light emission beauty device comprising:
a tubular body (11);
a light emission device comprising a lens (44) located at a side of one end of the tubular body (11) and a light source (41) located at the back of the lens (44);
a lighting circuit (5) being configured to activate the light source;
a controller (6) being configured to control the lighting circuit (5); and
a light-emitting switch (7) being configured to supply a light-emitting signal to the controller (6) in accordance with on operation,
**characterized in that** the controller (6) is configured to make the lighting circuit (5) activate the light source (41) in response to receiving the light-emitting signal continuously for a predetermined time.

2. The light emission beauty device as claimed in claim 1, wherein the controller (6) comprises a counter for counting the number of times of the light source's activation within a predetermined time to obtain a count value,
wherein the controller (6) is configured to lengthen time required to activate the light source (41) later in the activation, if the count value reaches a predetermined value.

3. The light emission beauty device as claimed in claim 1 or 2, wherein the controller (6) comprises a counter for counting the number of times of the light source's activation within a predetermined time to obtain a count value,
wherein the controller (6) is configured to prohibit the light source's activation for a given length of time, if the count value reaches a predetermined value.

4. The light emission beauty device as claimed in claim 1 or 2, wherein the lighting circuit (5) comprises a capacitor storing an electric charge for allowing the light source (41) to emit light and a voltage-detector for detecting voltage value of the capacitor,
wherein the controller (6) is configured to determine whether or not the voltage value detected with the voltage-detector just before the light source (41) is activated is in a predetermined range, the controller (6) being configured to prohibit the lighting circuit (5) from activating the light source (41) if the voltage value is out of the predetermined range.

5. The light emission beauty device as claimed in claim 3, wherein the lighting circuit (5) comprises a capacitor storing an electric charge for allowing the light source (41) to emit light a voltage-detector for detecting voltage value of the capacitor,
wherein the controller (6) is configured to determine whether or not the voltage value detected with the voltage-detector just before the light source (41) is activated is in a predetermined range, the controller (6) being configured to prohibit the lighting circuit (5) from activating the light source (41) if the voltage value is out of the predetermined range.

6. The light emission beauty device as claimed in claim 1, wherein the light emission device comprises an emission unit (4) which can be attached to and detached from the end of the tubular body (11),
wherein the tubular body (11) comprises a detection means for detecting whether or not the emission unit (4) is attached to the end of the tubular body.

7. The light emission beauty device as claimed in claim 6, wherein the lighting circuit (5) comprises a capacitor (13) storing an electric charge for allowing the light source (41) to emit light and a converter circuit (12) configured to supply an electric power to the capacitor (13),
wherein the converter circuit (12) comprises an electric source (120), a switching element (121), and a booster (122), the booster (122) boosting a voltage from the electric source (120) by means of on-off switching of the switching element (121) and supplying the boosted voltage to the capacitor (13),
wherein the tubular body (11) comprises a charging switch configured to supply a charging signal to the controller (6) in accordance with on operation,
wherein the controller (6) is configured to make the switching element (121) perform on-off switching in response to the charging signal if the detection means detects the emission unit (4),
wherein the controller(6) is configured to make the switching element (121) maintain off-state even if receiving the charging signal, if the detection means doesn't detect the emission unit.

8. The light emission beauty device as claimed in claim 6 or 7, wherein the controller (6) is configured to allow the lighting circuit (5) to activate the light source (41) in response to the light-emitting signal if the detection means detects the emission unit (4),
wherein the controller (6) is configured prohibit the lighting circuit (5) from activating the light source (41) even if receiving the light-emitting signal, the detection means doesn't detect the emission unit (4).

9. The light emission beauty device as claimed in claim 6 or 7, wherein the lighting circuit (5) comprises a capacitor (13) storing an electric charge for allowing the light source (41) to emit light, a converter circuit (12) configured to supply an electric power to the capacitor (13), and a current control element,
wherein the converter circuit (12) comprises an electric source (120), a switching element (121), and a booster (122), the booster (122) boosting a voltage from the electric source (120) by means of on-off switching of the switching element (121) and supplying the boosted voltage to the capacitor (13),
wherein the emission unit (4) comprises a first connector (42) electrically connected to the light source (41),
wherein the tubular body (11) comprises a second connector (24) electrically connected to the capacitor (13),
wherein the capacitor (13) and the light source (41) are electrically connected to each other when the second connector (24) joints the first connector (42),
wherein the current control element is located in an electric path between the capacitor (13) and the second connector and turns the electric path on or off,
wherein the controller (6) is configured to make the current control element turn off if the detection means doesn't detect the emission unit (4).

10. The light emission beauty device as claimed in claim 8, wherein the lighting circuit (5) comprises a capacitor (13) storing an electric charge for allowing the light source (41) to emit light, a converter circuit (12) configured to supply an electric power to the capacitor, and a current control element,
wherein the converter circuit (12) comprises an electric source (120), a switching element (121), and a booster (122), the booster (122) boosting a voltage from the electric source (120) by means of on-off switching of the switching element and supplying the boosted voltage to the capacitor (13),
wherein the emission unit (4) comprises a first connector (42) electrically connected to the light source (41),
wherein the tubular body comprises a second connector (24) electrically connected to the capacitor (13),
wherein the capacitor (13) and the light source (41) are electrically connected to each other when the second connector (24) joints the first connector,
wherein the current control element is located in an electric path between the capacitor (13) and the second connector (24) and turns the electric path on or off,
wherein the controller (6) is configured to make the current control element turn off if the detection means doesn't detect the emission unit (4).

11. The light emission beauty device as claimed in claim 1, wherein the lighting circuit (5) comprises a capacitor (13) storing an electric charge for allowing the light source (41) to emit light,
wherein the controller (6) comprises a counter for counting the number of times of the light source's activation to obtain a count value and a memory for storing a table having different voltage values corresponding to different count values, respectively, the controller being configured to correct a target value to be applied across the capacitor to a voltage value, corresponding to the count value obtained by the counter, in the table.

## Patentansprüche

1. Eine Lichtemissions-Kosmetikvorrichtung, die aufweist:
ein röhrenförmiges Rumpfteil (11);
eine Lichtemissions-Vorrichtung mit einer Linse (44), die an einer Seite eines Endes des röhrenförmigen Rumpfteils (11) angeordnet ist, und eine Lichtquelle (41), die an der Rückseite der Linse (44) angeordnet ist;
eine Lichtschaltung (5), die eingerichtet ist, die Lichtquelle zu aktivieren;
eine Steuerung (6), die eingerichtet ist, die Lichtschaltung (5) zu steuern; und
einen Licht emittierenden Schalter (7), der eingerichtet ist, gemäß einer Einschalt-Funktion ein Lichtemissionssignal an die Steuerung (6) zu liefern,
**dadurch gekennzeichnet, dass** die Steuerung (6) so eingerichtet ist, dass die Lichtschaltung (5) in Reaktion auf ein Empfangen des Lichtemissionssignals hin die Lichtquelle (41) für eine vorbestimmte Zeit kontinuierlich aktiviert.

2. Die Lichtemissions-Kosmetikvorrichtung gemäß Anspruch 1, wobei die Steuerung (6) einen Zähler zum Zählen der Anzahl der Aktivierungen der Lichtquelle innerhalb einer vorbestimmten Zeit erfasst, um einen Zählwert zu erhalten,
wobei die Steuerung (6) eingerichtet ist, eine Zeit, die für das Aktivieren der Lichtquelle (41) später in der Aktivierung erforderlich ist zu verlängern, wenn der Zählwert einen vorbestimmten Wert erreicht.

3. Die Lichtemissions-Kosmetikvorrichtung gemäß Anspruch 1 oder 2, wobei die Steuerung (6) einen Zähler zum Zählen der Anzahl der Aktivierungen der Lichtquelle innerhalb einer vorbestimmten Zeit erfasst, um einen Zählwert zu erhalten,
wobei die Steuerung eingerichtet ist, die Aktivierung der Lichtquelle für eine vorgegebene Zeitspanne zu verhindern, wenn der Zählwert einen vorbestimmten Wert erreicht.

4. Die Lichtemissions-Kosmetikvorrichtung gemäß Anspruch 1 oder 2, wobei die Lichtschaltung einen Kondensator umfasst, der eine elektrische Ladung speichert, um zu ermöglichen, dass die Lichtquelle (41) Licht ausstrahlt, und einen Spannungs-Detektor zum Erfassen eines Spannungswertes des Kondensators,
wobei die Steuerung (6) eingerichtet ist zu bestimmen, ob der mit dem Spannungsdetektor kurz vor der Aktivierung der Lichtquelle (41) erfasste Spannungswert in einem vorbestimmten Bereich liegt oder nicht, wobei die Steuerung (6) eingerichtet ist zu verhindern, dass die Lichtschaltung (5) die Lichtquelle (41) aktiviert, wenn der Spannungswert außerhalb des vorbestimmten Bereichs liegt.

5. Die Lichtemissions-Kosmetikvorrichtung gemäß Anspruch 3,
wobei die Lichtschaltung (5) umfasst: einen Kondensator, der eine elektrische Ladung speichert, um der Lichtquelle (41) zu ermöglichen, Licht auszustrahlen; einen Spannungs-Detektor zum Erfassen eines Spannungswertes des Kondensators;
wobei die Steuerung eingerichtet ist zu bestimmen, ob der mit dem Spannungsdetektor kurz vor der Aktivierung der Lichtquelle (41) erfasste Spannungswert in einem vorbestimmten Bereich liegt oder nicht, wobei die Steuerung (6) eingerichtet ist zu verhindern, dass die Lichtschaltung (5) die Lichtquelle (41) aktiviert, wenn der Spannungswert außerhalb des vorbestimmten Bereichs liegt.

6. Die Lichtemissions-Kosmetikvorrichtung gemäß Anspruch 1, wobei die Licht ausstrahlende Vorrichtung eine Sendeeinheit (4) umfasst, die an dem Ende des röhrenförmigen Rumpfteils (11) befestigt und von diesem abgenommen werden kann,
wobei das röhrenförmige Rumpfteil (11) eine Erfassungseinrichtung umfasst, mit der erfasst werden kann, ob die Sendeeinheit (4) an dem Ende des röhrenförmigen Rumpfteils befestigt ist oder nicht.

7. Die Lichtemissions-Kosmetikvorrichtung gemäß Anspruch 6, wobei die Lichtschaltung (5) einen Kondensator (13) umfasst, der eine elektrische Ladung speichert, um der Lichtquelle (41) zu ermöglichen, Licht auszustrahlen, und eine Wandlerschaltung (12), die eingerichtet ist, eine elektrische Leistung an den Kondensator (13) zu liefern,
wobei die Wandlerschaltung (12) eine elektrische Quelle (120), ein Schaltelement (121) und einen Spannungsverstärker (122) aufweist, wobei der Spannungsverstärker (122) eine Spannung von der elektrischen Quelle (120) mittels Ein-Aus-Schalten des Schaltelements (121) und Zuführen der verstärkten Spannung zum Kondensator (13),
wobei das röhrenförmige Rumpfteil einen Ladeschalter aufweist, der eingerichtet ist, gemäß Ein-Funktion ein Ladesignal an die Steuerung (6) zu liefern;
wobei die Steuerung (6) eingerichtet ist zu bewirken, dass das Schaltelement (121), wenn die Erfassungseinrichtung die Sendeeinheit (4) erfasst, auf das Ladesignal hin Ein-Aus-Schalten ausführt,
wobei die Steuerung (6) eingerichtet ist zu bewirken, dass das Schaltelement (121), wenn die Erfassungseinrichtung die Sendeeinheit nicht erfasst, Aus-Zustand beibehält, selbst wenn es das Ladesignal empfängt.

8. Die Lichtemissions-Kosmetikvorrichtung gemäß Anspruch 6 oder 7, wobei die Steuerung (6) eingerichtet ist, der Lichtschaltung (5), wenn die Erfassungseinrichtung die Sendeeinheit (4) erfasst, zu ermöglichen, die Lichtquelle (41) auf das Lichtemissionssignal hin zu aktivieren,
wobei die Steuerung (6) eingerichtet ist zu verhindern, dass die Lichtschaltung (5), wenn die Erfassungseinrichtung die Sendeeinheit (4) nicht erfasst, die Lichtquelle (41) aktiviert, selbst wenn sie das Lichtemissionssignal empfängt.

9. Die Lichtemissions-Kosmetikvorrichtung gemäß Anspruch 6 oder 7,
wobei die Lichtschaltung (5) umfasst: einen Kondensator (13), der eine elektrische Ladung speichert, um der Lichtquelle (41) ein Ausstrahlen von Licht zu ermöglichen; eine Wandlerschaltung (12), die eingerichtet ist, eine elektrische Leistung an den Kondensator (13) zu liefern; und ein Stromsteuerungselement;
wobei die Wandlerschaltung (12) eine elektrische Quelle (120), ein Schaltelement (121) und einen Spannungsverstärker (122) aufweist, wobei der Spannungsverstärker (122) eine Spannung von der elektrischen Quelle (120) mittels Ein-Aus-Schalten des Schaltelements (121) verstärkt, und Zuführen der verstärkten Spannung zum Kondensator (13),
wobei die Sendeeinheit (4) einen ersten Anschluss (42) umfasst, der elektrisch mit der Lichtquelle (41) verbunden ist,
wobei das röhrenförmige Rumpfteil (11) einen zweiten Anschluss (24) umfasst, der elektrisch mit dem Kondensator (13) verbunden ist,
wobei der Kondensator (13) und die Lichtquelle (41) elektrisch miteinander verbunden sind, wenn der zweite Anschluss (24) den ersten Anschluss (42) zusammenfügt,
wobei das Stromsteuerungselement in einem elektrischen Pfad zwischen dem Kondensator (13) und dem zweiten Anschluss angeordnet ist und den elektrischen Pfad ein- oder ausschaltet,
wobei die Steuerung (6) eingerichtet ist zu bewirken, dass das Stromsteuerungselement abschaltet, wenn die Erfassungseinrichtung die Sendeeinheit (4) nicht erfasst.

10. Die Lichtemissions-Kosmetikvorrichtung gemäß Anspruch 8,
wobei die Lichtschaltung (5) aufweist:
einen Kondensator (13), der eine elektrische Ladung speichert, um der Lichtquelle (41) ein Ausstrahlen von Licht zu ermöglichen; eine Wandlerschaltung (12), die eingerichtet ist, eine elektrische Leistung an den Kondensator (13) zu liefern; und eine Stromsteuerungselement;
wobei die Wandlerschaltung (12) eine elektrische Quelle (120), ein Schaltelement (121) und einen Spannungsverstärker (122) aufweist, wobei der Spannungsverstärker (122) eine Spannung von der elektrischen Quelle (120) mittels Ein- und Ausschalten des Schaltelements (121) verstärkt, und Zuführen der verstärkten Spannung zum Kondensator (13),
wobei die Sendeeinheit (4) einen ersten Anschluss (42) umfasst, der elektrisch mit der Lichtquelle (41) verbunden ist,
wobei das röhrenförmige Rumpfteil einen zweiten Anschluss (24) umfasst, der elektrisch mit dem Kondensator (13) verbunden ist,
wobei der Kondensator (13) und die Lichtquelle (41) elektrisch miteinander verbunden sind, wenn der zweite Anschluss (24) den ersten Anschluss (42) zusammenfügt,
wobei das Stromsteuerungselement in einem elektrischen Pfad zwischen dem Kondensator (13) und dem zweiten Anschluss (24) angeordnet ist und den elektrischen Pfad ein- oder ausschaltet,
wobei die Steuerung (6) eingerichtet ist zu bewirken, dass das Stromsteuerungselement abschaltet, wenn die Erfassungseinrichtung die Sendeeinheit (4) nicht erfasst.

11. Die Lichtemissions-Kosmetikvorrichtung gemäß Anspruch 1, wobei die Lichtschaltung (5) einen Kondensator (13) aufweist, der eine elektrische Ladung speichert, um der Lichtquelle (41) ein Ausstrahlen von Licht zu ermöglichen;
wobei die Steuerung (6) einen Zähler zum Zählen der Anzahl der Aktivierungen der Lichtquelle innerhalb einer vorbestimmten Zeit erfasst, um einen Zählwert zu erhalten, und einen Speicher zum Speichern einer Tabelle, die verschiedene, jeweils unterschiedlichen Zählwerten entsprechende Spannungswerte enthält,
wobei die Steuerung eingerichtet ist, einen Zielwert zu korrigieren, der über den Kondensator auf einen Spannungswert anzuwenden ist entsprechend dem Zählwert, der durch den Zähler in der Tabelle erhalten wird.

## Revendications

1. Dispositif de beauté à émission de lumière comprenant :
un corps tubulaire (11) ;
un dispositif d'émission de lumière comprenant une lentille (44) située d'un côté d'une extrémité du corps tubulaire (11) et une source de lumière (41) située à l'arrière de la lentille (44) ;
un circuit d'éclairage (5) configuré pour activer la source de lumière ;
un contrôleur (6) configuré pour commander le circuit d'éclairage (5) ; et
un commutateur d'émission de lumière (7) configuré pour fournir un signal d'émission de lumière au contrôleur (6) conformément à une opération de mise en marche,
**caractérisé en ce que** le contrôleur (6) est configuré pour amener le circuit d'éclairage (5) à activer la source de lumière (41) en réponse à la réception du signal d'émission de lumière continûment pendant un temps prédéterminé.

2. Dispositif de beauté à émission de lumière selon la revendication 1, dans lequel le contrôleur (6) comprend un compteur pour compter le nombre d'activations de la source de lumière dans les limites d'un temps prédéterminé pour obtenir une valeur de comptage,
dans lequel le contrôleur (6) est configuré pour allonger un temps nécessaire pour activer la source de lumière (41) plus tard au cours de l'activation, si la valeur de comptage atteint une valeur prédéterminée.

3. Dispositif de beauté à émission de lumière selon la revendication 1 ou 2, dans lequel le contrôleur (6) comprend un compteur pour compter le nombre d'activations de la source de lumière dans les limites d'un temps prédéterminé pour obtenir une valeur de comptage,
dans lequel le contrôleur (6) est configuré pour interdire l'activation de la source de lumière pendant une période de temps donnée, si la valeur de comptage atteint une valeur prédéterminée.

4. Dispositif de beauté à émission de lumière selon la revendication 1 ou 2, dans lequel le circuit d'éclairage (5) comprend un condensateur stockant une charge électrique pour permettre à la source de lumière (41) d'émettre une lumière et un détecteur de tension pour détecter une valeur de tension du condensateur,
dans lequel le contrôleur (6) est configuré pour déterminer si, oui ou non, la valeur de tension détectée par le détecteur de tension juste avant que la source de lumière (41) soit activée est dans une plage prédéterminée, le contrôleur (6) étant configuré pour interdire l'activation par le circuit d'éclairage (5) de la source de lumière (41) si la valeur de tension est en-dehors de la plage prédéterminée.

5. Dispositif de beauté à émission de lumière selon la revendication 3, dans lequel le circuit d'éclairage (5) comprend un condensateur stockant une charge électrique pour permettre à la source de lumière (41) d'émettre une lumière et un détecteur de tension pour détecter une valeur de tension du condensateur,
dans lequel le contrôleur (6) est configuré pour déterminer si, oui ou non, la valeur de tension détectée par le détecteur de tension juste avant que la source de lumière (41) soit activée est dans une plage prédéterminée, le contrôleur (6) étant configuré pour interdire au circuit d'éclairage (5) d'activer la source de lumière (41) si la valeur de tension est en-dehors de la plage prédéterminée.

6. Dispositif de beauté à émission de lumière selon la revendication 1, dans lequel le dispositif d'émission de lumière comprend une unité d'émission (4) qui peut être attachée à l'extrémité du corps tubulaire (11) et détachée de celle-ci,
dans lequel le corps tubulaire (11) comprend des moyens de détection pour détecter si, oui ou non, l'unité d'émission (4) est attachée à l'extrémité du corps tubulaire.

7. Dispositif de beauté à émission de lumière selon la revendication 6, dans lequel le circuit d'éclairage (5) comprend un condensateur (13) stockant une charge électrique pour permettre à la source de lumière (41) d'émettre une lumière et un circuit convertisseur (12) configuré pour fournir une alimentation électrique au condensateur (13),
dans lequel le circuit convertisseur (12) comprend une source électrique (120), un élément de commutation (121), et un suramplificateur (122), le suramplificateur (122) suramplifiant une tension provenant de la source électrique (120) au moyen d'une commutation tout ou rien de l'élément de commutation (121) et fournissant la tension suramplifiée au condensateur (13),
dans lequel le corps tubulaire (11) comprend un commutateur de charge configuré pour fournir un signal de charge au contrôleur (6) conformément à une opération de mise en marche,
dans lequel le contrôleur (6) est configuré pour amener l'élément de commutation (121) à exécuter une commutation tout ou rien en réponse au signal de charge si les moyens de détection détectent l'unité d'émission (4),
dans lequel le contrôleur (6) est configuré pour amener l'élément de commutation (121) à maintenir un état désactivé même si le signal de charge est reçu, si les moyens de détection ne détectent pas l'unité d'émission.

8. Dispositif de beauté à émission de lumière selon la revendication 6 ou 7, dans lequel le contrôleur (6) est configuré pour permettre au circuit d'éclairage (5) d'activer la source de lumière (41) en réponse au signal d'émission de lumière si les moyens de détection détectent l'unité d'émission (4),
dans lequel le contrôleur (6) est configuré pour interdire au circuit d'éclairage (5) d'activer la source de lumière (41) même si le signal d'émission de lumière est reçu, si les moyens de détection ne détectent pas l'unité d'émission (4).

9. Dispositif de beauté à émission de lumière selon la revendication 6 ou 7, dans lequel le circuit d'éclairage (5) comprend un condensateur (13) stockant une charge électrique pour permettre à la source de lumière (41) d'émettre une lumière, un circuit convertisseur (12) configuré pour fournir une alimentation électrique au condensateur (13), et un élément de commande de courant,
dans lequel le circuit convertisseur (12) comprend une source électrique (120), un élément de commutation (121), et un suramplificateur (122), le suramplificateur (122) suramplifiant une tension provenant de la source électrique (120) au moyen d'une commutation tout ou rien de l'élément de commutation (121) et fournissant la tension suramplifiée au condensateur (13),
dans lequel l'unité d'émission (4) comprend un premier connecteur (42) connecté électriquement à la source de lumière (41),
dans lequel le corps tubulaire (11) comprend un deuxième connecteur (24) connecté électriquement au condensateur (13),
dans lequel le condensateur (13) et la source de lumière (41) sont connectés électriquement l'un à l'autre lorsque le deuxième connecteur (24) est joint au premier connecteur (42),
dans lequel l'élément de commande de courant est situé dans un trajet électrique entre le condensateur (13) et le deuxième connecteur et ouvre ou ferme le trajet électrique,
dans lequel le contrôleur (6) est configuré pour provoquer l'ouverture de l'élément de commande de courant si les moyens de détection ne détectent pas l'unité d'émission (4).

10. Dispositif de beauté à émission de lumière selon la revendication 8, dans lequel le circuit d'éclairage (5) comprend un condensateur (13) stockant une charge électrique pour permettre à la source de lumière (41) d'émettre une lumière, un circuit convertisseur (12) configuré pour appliquer une alimentation électrique au condensateur, et un élément de commande de courant,
dans lequel le circuit convertisseur (12) comprend une source électrique (120), un élément de commutation (121), et un suramplificateur (122), le suramplificateur (122) suramplifiant une tension provenant de la source électrique (120) au moyen d'une commutation tout ou rien de l'élément de commutation et fournissant la tension suramplifiée au condensateur (13),
dans lequel l'unité d'émission (4) comprend un premier connecteur (42) connecté électriquement à la source de lumière (41),
dans lequel le corps tubulaire comprend un deuxième connecteur (24) connecté électriquement au condensateur (13),
dans lequel le condensateur (13) et la source de lumière (41) sont connectés électriquement l'un à l'autre lorsque le deuxième connecteur (24) est joint au premier connecteur,
dans lequel l'élément de commande de courant est situé dans un trajet électrique entre le condensateur (13) et le deuxième connecteur (24) et ferme ou ouvre le trajet électrique,
dans lequel le contrôleur (6) est configuré pour amener l'élément de commande de courant à s'ouvrir si les moyens de détection ne détectent pas l'unité d'émission (4).

11. Dispositif de beauté à émission de lumière selon la revendication 1, dans lequel le circuit d'éclairage (5) comprend un condensateur (13) stockant une charge électrique pour permettre à la source de lumière (41) d'émettre une lumière,
dans lequel le contrôleur (6) comprend un compteur pour compter le nombre d'activations de la source de lumière pour obtenir une valeur de comptage et une mémoire pour mémoriser une table comportant différentes valeurs de tension correspondant à différentes valeurs de comptage, respectivement, le contrôleur étant configuré pour corriger une valeur cible à appliquer aux bornes du condensateur à une valeur de tension, correspondant à la valeur de comptage obtenue par le compteur, dans la table.
